(19) Europäisches Patentamt
European Patent Office
Office européen des brevets

(11) Veröffentlichungsnummer : **0 422 493 B1**

(12) **EUROPÄISCHE PATENTSCHRIFT**

(45) Veröffentlichungstag der Patentschrift : **29.12.93 Patentblatt 93/52**

(51) Int. Cl.$^5$ : **C07C 327/24,** C07C 327/28, C07C 327/58

(21) Anmeldenummer : **90118944.9**

(22) Anmeldetag : **04.10.90**

(54) **Verfahren zur Herstellung von Cyclohexantrioncarbonsäurethiolestern und neue Zwischenprodukte.**

(30) Priorität : **13.10.89 DE 3934205**

(43) Veröffentlichungstag der Anmeldung : **17.04.91 Patentblatt 91/16**

(45) Bekanntmachung des Hinweises auf die Patenterteilung : **29.12.93 Patentblatt 93/52**

(84) Benannte Vertragsstaaten : **AT BE CH DE DK ES FR GB GR IT LI NL SE**

(56) Entgegenhaltungen :
**EP-A- 0 125 094**
**EP-A- 0 293 817**
**US-A- 2 458 075**

(73) Patentinhaber : **BASF Aktiengesellschaft**
**Carl-Bosch-Strasse 38**
**D-67063 Ludwigshafen (DE)**

(72) Erfinder : **Kast, Juergen, Dr.**
**Kastanienstrasse 24**
**W-6737 Boehl-Iggelheim (DE)**
Erfinder : **Schubert, Juergen, Dr.**
**Am Oberen Luisenpark 2**
**W-6800 Mannheim 1 (DE)**
Erfinder : **Kaczmarek, Reinhard, Dr.**
**Am Stuempel 3**
**W-6719 Bobenheim (DE)**

EP 0 422 493 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von Cyclohexantrioncarbonsäurethiolestern der allgemeinen Formel I

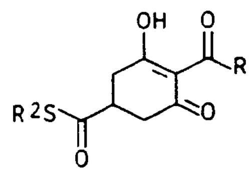

I

in der
$R^1$ für $C_1$-$C_{20}$-Alkyl, $C_2$-$C_{20}$-Alkenyl, $C_2$-$C_{20}$-Alkinyl oder $C_3$-$C_6$-Cycloalkyl, jeweils unsubstituiert oder durch Halogen, $C_1$-$C_4$-Alkyloxy, $C_1$-$C_4$-Alkylthio substituiert, Benzyl oder Phenyl jeweils unsubstituiert oder substituiert durch Halogen, Cyano, $C_1$-$C_4$-Alkyl, $C_1$-$C_4$-Alkoxy, $C_1$-$C_4$-Halogenalkyl oder Nitro und
$R^2$ für Wasserstoff, $C_1$-$C_6$-Alkyl, $C_2$-$C_6$-Alkenyl, $C_2$-$C_6$-Alkinyl und $C_3$-$C_6$-Cycloalkyl, jeweils unsubstituiert oder durch $C_1$-$C_4$-Alkoxy, $C_1$-$C_4$-Alkylthio, $C_1$-$C_4$-Dialkylamino, Hydroxy, Halogen substituiert; Benzyl oder Phenyl, jeweils unsubstituiert oder substituiert durch Halogen, Cyano, $C_1$-$C_4$-Alkyl, $C_1$-$C_4$-Alkoxy oder Nitro stehen.

Weiterhin werden erfindungsgemäß neue Zwischenprodukte zur Verfügung gestellt.

Cyclohexantrioncarbonsäurethiolester I sind hochwirksame Bioregulatoren wie aus EP-A 293 817 hervorgeht. Zur Darstellung von Thiolestern stehen verschiedene Möglichkeiten zur Verfügung. Einige Methoden sind im folgenden dargestellt.

Weg A:

Aus Carbonsäuren mit Thiolen unter Carbonylaktivierung. Als aktivierende Reagenzien kommen z.B. Diphenylphosphinsäure-chlorid (Monatsh. Chem. 110, 759 (1979)), Phosphorsäure-cyaniddiethylester (Tetrahedron Lett. 1973, 1595)). Carbonyl-bis-imidazolid und Carbonyl-bis-triazolid (Angew. Chem. 89, 251 (1977); J. Am. Chem. Soc. 93, 1419 (1971)) in Frage.

Weg B:

Aus Carbonsäurehalogeniden, -anhydriden und -estern mit Thiolen bzw. Thiolaten gegebenenfalls in Gegenwart einer Base (Houben-Weyl, Methoden der organischen Chemie, Bd 9, Seiten 753-760, 1955).

Weg C:

Durch radikalische Substitution an Aldehyden mit organischen Disulfiden unter UV-Licht-Katalyse bzw. mit Radikalstartern wie Azobisisobutyronitril (Bull. Chem. Soc. Jpn. 53, 1982 (1980)).

Alle diese Verfahren weisen einen oder mehrere schwerwiegende Nachteile auf. Gemäß Weg A werden teure oder schwer zugängliche Aktivierungsreagenzien, die eine kommerzielle Nutzung ausschließen, verwendet. Gemäß Weg C wird die Aldehydkomponente in hohem Überschuß bzw. als Lösungsmittel verwendet, wodurch das Verfahren bei teuren Aldehyden unwirtschaftlich ist.

Weg B, die Umsetzung mit Säurehalogeniden bzw. -anhydriden, ist eine Standardmethode zur Herstellung von Thiolestern. Bei den vorliegenden Cyclohexantrionen besteht aber die Gefahr, daß mit den gängigen Halogenierungsreagenzien, wie Thionylchlorid, Phosphoroxychlorid, Sulfurylchlorid, Oxalylchlorid oder Phosgen, auch die vinylische OH-Funktion gegen Halogen z.B. Chlor, Brom, ausgetauscht wird.

Aus der Literatur ist ferner bekannt, daß sich Mercaptane in Gegenwart von Chlorwasserstoff an Nitrile addieren. Die dabei entstehenden Imino-thiocarbonsäure-S-ester können zu Thiocarbonsäure-S-estern hydrolysiert werden (A. Pinner, Die Iminoether, S. 80 Verlag R. Oppenheim, Berlin 1892). Wegen der konkurrierenden Hydrolyse zu Carbonsäureamiden bzw. Carbonsäuren werden aber im allgemeinen nur mäßige Ausbeuten von ca. 10 bis 25 % erzielt (siehe z.B. US 2 458 075 (1946) Philipps Petrol Co, Erfinder: Ch. M. Himel, CA 43, 3444 (1949) oder Ber. Dt. Chem. Ges. 69, 2352 (1936)), weshalb dieser Reaktionsfolge keine präparative Bedeutung zugemessen wird (Houben-Weyl, Methoden der organischen Chemie, E5/I S. 876, 1985).

Der Erfindung lag nun die Aufgabe zugrunde, ein Verfahren zur Herstellung der eingangs definierten Cyclohexantrioncarbonsäurethiolester I zu finden, das einfach und im technischen Maßstab durchführbar ist,

das Wertprodukt I in guten Ausbeuten liefert und wenige Verfahrensstufen erfordert.

Demgemäß wurde ein Verfahren zur Herstellung von Cyclohexantrioncarbonsäurethiolestern der Formel I

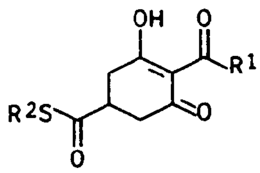

I

in der

$R^1$ für $C_1$-$C_{20}$-Alkyl, $C_2$-$C_{20}$-Alkenyl, $C_2$-$C_{20}$-Alkinyl oder $C_3$-$C_6$-Cycloalkyl, jeweils unsubstituiert oder durch Halogen, $C_1$-$C_4$-Alkyloxy, $C_1$-$C_4$-Alkylthio substituiert, Benzyl oder Phenyl jeweils unsubstituiert oder substituiert durch Halogen, Cyano, $C_1$-$C_4$-Alkyl, $C_1$-$C_4$-Alkoxy, $C_1$-$C_4$-Halogenalkyl oder Nitro und

$R^2$ für Wasserstoff, $C_1$-$C_6$-Alkyl, $C_2$-$C_6$-Alkenyl, $C_2$-$C_6$-Alkinyl und $C_3$-$C_6$-Cycloalkyl, jeweils unsubstituiert oder durch $C_1$-$C_4$-Alkoxy, $C_1$-$C_4$-Alkylthio, $C_1$-$C_4$-Dialkylamino, Hydroxy, Halogen substituiert; Benzyl oder Phenyl, jeweils unsubstituiert oder substituiert durch Halogen, Cyano, $C_1$-$C_4$-Alkyl, $C_1$-$C_4$-Alkoxy oder Nitro

stehen; gefunden, das dadurch gekennzeichnet ist, daß man in einer ersten Stufe eine Verbindung der allgemeinen Formel II

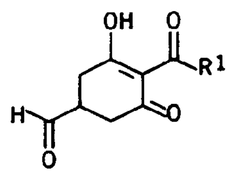

II

in der $R^1$ die oben angegebene Bedeutung hat, mit Hydroxylamin oder Hydroxylamin-O-sulfonsäure in einem inerten Lösungsmittel bei Temperaturen von 0 bis 150°C zu Verbindungen der allgemeinen Formel III

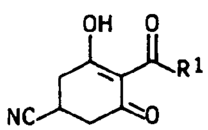

III,

in der $R^1$ die oben angegebene Bedeutung hat, umsetzt,

in einer zweiten Stufe diese Cyanoverbindungen der Formel III dann mit einem Mercaptan der allgemeinen Formel IV

$R^2$-SH IV

in der $R^2$ die oben angegebene Bedeutung hat, in Gegenwart einer Säure HX, zu einer Verbindung der allgemeinen Formel V

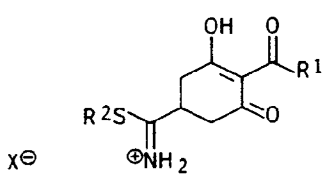

V

in der $R^1$ und $R^2$ die oben angegebene Bedeutung haben und X für einen Säurerest steht, umsetzt und

in einer dritten Stufe die Verbindungen der Formel V durch Hydrolyse in die Verbindungen der allgemeinen Formel I überführt.

Die Reaktionssequenz des erfindungsgemäßen Verfahrens ist anhand des folgenden Schemas dargestellt; wobei für $R^1$ Methyl, für $R^2$ Ethyl und als Säure in der zweiten Verfahrensstufe Chlorwasserstoff gewählt wurde.

Reaktionsschema:

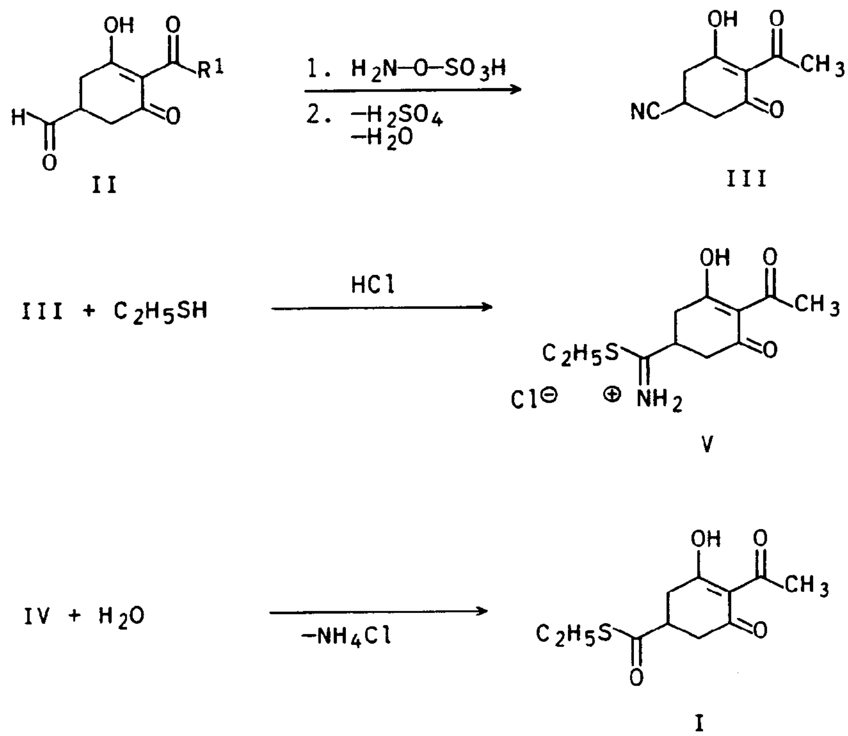


II III V I

In der ersten Verfahrensstufe wird die Formylverbindung II, die wie in DE-A 233 568 beschrieben, zugänglich ist, mit Hydroxylamin-O-sulfonsäure oder Hydroxylamin unter Kondensationsbedingungen in einem inerten Lösungsmittel bei Temperaturen von 0 bis 150, insbesondere 20 bis 80°C, umgesetzt. Als besonders günstig hat es sich erwiesen, die Umsetzung mit Hydroxylamin-O-sulfonsäure in Wasser, z.B. mit 1 bis 100 Gew.-Teilen Wasser, bezogen auf die Formylverbindung II, vorzunehmen.

Die Umsetzung kann in homogener oder heterogener wäßriger Phase, ggf. unter Zusatz eines Puffers, durchgeführt werden, wobei der pH-Bereich im allgemeinen bei 1 bis 9, insbesondere 3 bis 8, liegt.

Zweckmäßigerweise verfährt man in der Weise, daß man die Formylverbindung II in Wasser suspendiert oder durch Zusatz eines wassermischbaren organischen Lösungsmittels wie z.B. Methanol, Ethanol in Lösung bringt. Es ist auch möglich, den Ausgangsstoff II in wassernichtmischbaren Lösungsmitteln wie Ethern, z.B. Diethylether; chlorierten Kohlenwasserstoffen wie Methylenchlorid, Chloroform, Dichlorethan; Estern wie Essigester oder aromatischen Verbindungen wie Benzol, Toluol, Xylole zu lösen. Zu der Mischung aus Lösungsmittel und Ausgangsstoff II gibt man die Hydroxylamin-O-sulfonsäure in fester Form oder als wäßrige Lösung.

Wenn die Umsetzung in heterogener Phase vorgenommen wird, ist es vorteilhaft für eine intensive Durchmischung der Phasen zu sorgen.

Um eine vollständige Umsetzung zur Cyanoverbindung III zu erreichen, empfiehlt es sich die Reaktionskomponenten in äquimolaren Mengen einzusetzen. Aus verfahrenstechnischen Gründen kann es ratsam sein die Formylverbindung II oder die Hydroxylamin-O-sulfonsäure im Überschuß z.B. von 1 bis 100 Mol.% einzusetzen. Vorzugsweise verwendet man die Hydroxylamin-O-sulfonsäure in einem Überschuß von 10 bis 20 Mol.%.

In der Regel sind Reaktionstemperaturen bis 60°C, insbesondere 20 bis 40°C, für eine vollständige Umsetzung ausreichend.

In manchen Fällen kann es vorteilhaft sein, die Reaktion durch Zugabe katalytischer Mengen einer Base zu beschleunigen.

Geeignete Basen sind z.B. Alkalihydroxide wie NaOH oder KOH, Ammoniumhydroxid, Erdalkalihydroxide wie Magnesiumhydroxid oder Calciumhydroxid, Alkalicarbonate oder Alkalihydrogencarbonate wie Kaliumcarbonat oder Natriumhydrogencarbonat. Üblicherweise können Basenmengen von 0 bis 3 Äquivalenten, bezogen auf den Ausgangsstoff II, verwendet werden.

Die Umsetzung kann bei Normaldruck, Über- oder Unterdruck, kontinuierlich oder diskontinuierlich nach den dafür üblichen Techniken durchgeführt werden.

Die Isolierung der Cyanocyclohexenonverbindung III aus dem rohen Reaktionsgemisch kann in üblicher

Weise, z.B. durch Extraktion oder Filtration erfolgen.

Anstelle von Hydroxylamin-O-sulfonsäure kann man auch Hydroxylamin mit dem Ausgangsstoff II umsetzen und anschließend in üblicher Weise, z.B. durch erhitzen mit Essigsäureanhydrid oder Thionylchlorid bei Temperaturen von ca. 80 bis 150°C Wasser eliminieren.

In der zweiten Verfahrensstufe wird unter Wasserausschluß das Cyanocyclohexantrion III mit einem Mercaptan $R^2$-SH IV in Gegenwart einer wasserfreien Säure umgesetzt. Solche Säuren sind z.B. Chlorwasserstoff, Bromwasserstoffsäure, Schwefelsäure, Perchlorsäure oder Tetrafluorborsäure oder starke Carbonsäuren wie Trifluoressigsäure. Besonders bevorzugt sind Bromwasserstoff und insbesondere Chlorwasserstoff.

Um eine vollständige Umsetzung zu erreichen, ist es sinnvoll das Mercaptan IV und die Cyanoverbindung III in äquimolaren Mengen einzusetzen. Aus verfahrenstechnischen Gründen kann es vorteilhaft sein, eine der beiden Komponenten im Überschuß einzusetzen. Vorzugsweise wird das Mercaptan in einem Überschuß von 0 bis 200 Mol.%, bevorzugt von 5 bis 100 Mol.% verwendet.

Bevorzugte Reste $R^2$ im Mercaptan sind: Methyl, Ethyl, Propyl, i-Propyl, t-Butyl, n-Hexyl, Allyl, Butenyl, Methoxyethyl, 3-Chlorpropyl, 2-Dimethylaminoethyl, 2-Hydroxyethyl, Cyclohexyl, Benzyl, 4-Methylbenzyl, 4-Chlorbenzyl, Phenyl, p-Tolyl, 4-Chlorphenyl, 4-Methoxyphenyl, 3-Nitrophenyl.

Die Umsetzung der Cyanocyclohexantrionverbindung III mit dem Mercaptan IV wird zweckmäßigerweise in einem aprotischen organischen Verdünnungsmittel durchgeführt. Hierfür geeignet sind z.B. Ether wie Diethylether, Tetrahydrofuran, Dioxan; Kohlenwasserstoffe wie Pentan, Hexan, Cyclohexan, Petrolether, Benzol, Toluol; halogenierte Kohlenwasserstoffe wie Methylenchlorid, Chloroform, Tetrachlorkohlenstoff, 1,2-Dichlorethan.

Die Umsetzung kann in einem Temperaturbereich von -20°C bis 100°C vorzugsweise zwischen 0°C und +40°C bei Normaldruck, Über- oder Unterdruck nach den dafür üblichen Techniken durchgeführt werden.

Zweckmäßigerweise führt man die Umsetzung in der Form durch, daß man die Cyanocyclohexantrionverbindung III zusammen mit dem Mercaptan IV im Verdünnungsmittel unter weitgehendem Ausschluß von Wasser vorlegt und die Säure bei tiefer Temperatur, z.B. 0°C hinzutropft oder gasförmig einleitet. Zur Vervollständigung der Umsetzung kann dann auf höhere Temperaturen, z.B. 20 bis 40°C erwärmt werden.

Die Isolierung des Imido-thioester-salzes V, welches sich im allgemeinen kristallin oder als Öl abscheidet, kann in an sich bekannter Weise, z.B. durch Filtration oder Extraktion, erfolgen. Ist man an der Isolierung der Zwischenprodukte V nicht interessiert, so können diese durch wäßrige Extraktion bei gleichzeitiger Hydrolyse in die Endprodukte I überführt werden.

Die Hydrolyse der Zwischenprodukte V kann in Wasser, vorzugsweise in Gegenwart einer Säure oder bevorzugt in verdünnter wäßriger Mineralsäure z.B. Salzsäure, Schwefelsäure, Phosphorsäure oder verdünnten Carbonsäuren wie Essigsäure oder Ameisensäure vorgenommen werden.

Die Säuremenge ist nicht besonders kritisch und liegt im allgemeinen bei 0 bis 100 Gew.-teile Säure, bezogen auf V. Die Konzentration der Säure liegt zwischen 0 und 20 Gew.-%, bevorzugt werden wäßrige Lösungen mit 0 bis 10 Gew.-% Säure verwendet. Bevorzugt liegt der pH-Wert des Reaktionsgemisches bei 1 bis 7, insbesondere unter 2.

Die Reaktionstemperaturen sind ebenfalls wenig kritisch und liegen in der Regel bei 0 bis 100, insbesondere 10 bis 40°C.

Da die Thiolester der allgemeinen Formel I, in Wasser nicht löslich sind (Löslichkeit 100 mg/l), fallen sie nach der Hydrolyse von V als Öl oder Feststoff an. Durch Zugabe eines mit Wasser nichtmischbaren Lösungsmittels wie Ether z.B. Diethylether; Ester z.B. Essigester; chlorierte Kohlenwasserstoffe z.B. Methylenchlorid, Chloroform, Tetrachlorkohlenstoff, Dichlorethan; aromatische Kohlenwasserstoffe z.B. Benzol, Toluol, Xylole kann die Hydrolyse in einem Zweiphasensystem ablaufen, was vorteilhaft dazu führt, daß das Reaktionsprodukt I direkt aus der organischen Phase isoliert werden kann. Natürlich ist es auch möglich, erst im Anschluß an die Hydrolyse dem Reaktionsgemisch organische Lösungsmittel zur Isolierung von I zuzusetzen.

Im übrigen weist das Verfahren, welches auch als Eintopfverfahren ohne Isolierung der beschriebenen Zwischenprodukte III und V durchgeführt werden kann, keine verfahrenstechnischen Besonderheiten auf.

Erfindungsgemäß werden neue Zwischenprodukte V zur Verfügung gestellt.

Die Cyclohexantrionimidothioestersalze V werden durch folgende Formel charakterisiert:

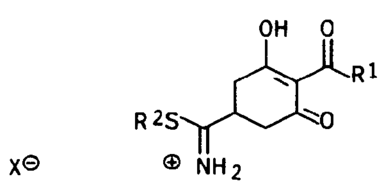

V,

in der

$R^1$ für $C_1$-$C_{20}$-Alkyl, $C_2$-$C_{20}$-Alkenyl, $C_2$-$C_{20}$-Alkinyl oder $C_3$-$C_6$-Cycloalkyl, jeweils unsubstituiert oder durch Halogen, $C_1$-$C_4$-Alkyloxy, $C_1$-$C_4$-Alkylthio substituiert, Benzyl oder Phenyl jeweils unsubstituiert oder substituiert durch Halogen, Cyano, $C_1$-$C_4$-Alkyl, $C_1$-$C_4$-Alkoxy, $C_1$-$C_4$-Halogenalkyl oder Nitro;

$R^2$ für Wasserstoff, $C_1$-$C_6$-Alkyl, $C_2$-$C_6$-Alkenyl, $C_2$-$C_6$-Alkinyl und $C_3$-$C_6$-Cycloalkyl, jeweils unsubstituiert oder durch $C_1$-$C_4$-Alkoxy, $C_1$-$C_4$-Alkylthio, $C_1$-$C_4$-Dialkylamino, Hydroxy, Halogen substituiert; Benzyl oder Phenyl, jeweils unsubstituiert oder substituiert durch Halogen, Cyano, $C_1$-$C_4$-Alkyl, $C_1$-$C_4$-Alkoxy oder Nitro und

X für einen Säurerest aus der Gruppe Chlorid, Bromid, Sulfat, Phosphat, Tetrafluoroborat, Perchlorat und Trifluoracetat

stehen.

Im Hinblick auf die biologische Wirksamkeit der Endprodukte I haben in Formel V die Substituenten $R^1$ und $R^2$ sowie X die folgende bevorzugte Bedeutung:

$R^1$
- $C_1$-$C_6$-Alkyl wie Methyl, Ethyl, Propyl, Isopropyl, n-Butyl, iso-Butyl, s-Butyl, t-Butyl, n-Pentyl, n-Hexyl
- $C_3$- bis $C_6$-Cycloalkyl wie Cyclopropyl, Cyclopentyl, Cyclohexyl
- $C_2$- bis $C_6$-Alkoxyalkyl, z.B. $C_1$-$C_4$-Alkoxy-$C_1$-$C_4$-Alkyl wie Methoxymethyl, Ethoxymethyl, Propyloxymethyl, 2-Methoxyethyl, 2-Ethoxyethyl, 2-Propyloxyethyl, Methoxypropyl, Ethoxypropyl,
- $C_2$- bis $C_6$-Alkylthioalkyl z.B. Methylthiomethyl, Ethylthiomethyl, Methylthioethyl, Ethylthioethyl, Methylthiopropyl, Ethylthiopropyl,
- Benzyl oder Phenyl, wobei die aromatischen Kerne ggf. ein- bis dreifach durch Halogen wie Chlor, Brom, Fluor, durch Cyano, Nitro, $C_1$-$C_4$-Alkyl wie Methyl, Ethyl, Propyl oder Butyl, $C_1$-$C_4$-Alkoxy wie Methoxy, Ethoxy, Propoxy oder Butoxy; $C_1$-$C_4$-Halogenalkyl wie Trifluormethyl, Difluorchlormethyl, Difluormethyl, Trichlormethyl, Tetrafluorethyl substituiert sind,

$R^2$
- $C_1$-$C_6$-Alkyl wie Methyl, Ethyl, Propyl, iso-Propyl, n-Butyl, iso-Butyl, sec.-Butyl, n-Pentyl und n-Hexyl,
- $C_3$-$C_5$-Alkenyl wie Allyl, But-2-enyl oder But-3-enyl, Pent-2-enyl,
- $C_3$- oder $C_4$-Alkinyl wie Propargyl, But-2-inyl, But-3-inyl,
- $C_3$-$C_6$-Cycloalkyl wie für $R^1$ genannt,
- $C_2$-$C_6$-Alkoxyalkyl oder Alkylthioalkyl jeweils wie für $R^1$ genannt,
- Phenyl oder Benzyl, wobei die aromatischen Kerne wie für $R^1$ genannt substituiert sein können;

X
- Chlorid oder Bromid

Besonders bevorzugt stehen $R^1$ für $C_1$-$C_4$-Alkyl, $C_3$-$C_6$-Cycloalkyl oder Phenyl, $R^2$ für $C_1$-$C_6$-Alkyl, $C_3$-$C_4$-Alkenyl, $C_3$-$C_4$-Alkinyl, $C_2$-$C_6$-Alkoxyalkyl oder Benzyl und X für Chlorid.

Beispiel 1.1

3,5-Dioxo-4-acetyl-cyclohexancarbonsäurenitril

19,3 g (0,11 Mol) 5-Formyl-2-acetyl-cyclohexan-1,3-dion wurden in 200 ml destilliertem Wasser vorgelegt und bei Raumtemperatur mit 14,7 g (0,13 Mol) Hydroxylamin-O-sulfonsäure versetzt. Anschließend wurde 17 h bei Raumtemperatur nachgerührt. Der Niederschlag wurde abgesaugt, mit Wasser gewaschen und getrocknet. Es wurden 11,5 g (61 % d. Th.) 3,5-Dioxo-4-acetyl-cyclohexancarbonsäurenitril erhalten (Fp. 94 bis 97°C).

Beispiel 1.2

3,5-Dioxo-4-propionyl-cyclohexancarbonsäurenitril

30,7 g (0,16 Mol) 5-Formyl-2-propionyl-cyclohexan-1,3-dion wurden in 150 ml destilliertem Wasser vorgelegt und bei Raumtemperatur mit 21,0 g (0,19 Mol) Hydroxylamin-O-sulfonsäure versetzt. Anschließend wurde über Nacht bei Raumtemperatur nachgerührt. Der Niederschlag wurde abgesaugt, mit Wasser gewaschen und getrocknet. Es wurden 23,8 g (71 % d. Th.) 3,5-Dioxo-4-propionyl-cyclohexancarbonsäurenitril erhalten (Fp. 70 bis 74°C).

Beispiel 1.5

3,5-Dioxo-4-palmitoyl-cyclohexancarbonsäurenitril

43 g (0,12 Mol) 5-Formyl-2-palmitoyl-cyclohexan-1,3-dion wurden in 150 ml destilliertem Wasser suspendiert. Hierzu gab man 15,7 g (0,14 Mol) Hydroxylamin-O-sulfonsäure, gelöst in 50 ml Wasser. Das heterogene

Reaktionsgemisch wurde über Nacht bei Raumtemperatur gerührt. Der erhaltene Feststoff wurde abgesaugt, mit Wasser gewaschen und getrocknet. Es wurden 37,7 g (86 % d. Th.) 3,5-Dioxo-4-palmitoyl-cyclohexancarbonsäurenitril isoliert (Fp. 66 bis 69°C).

Die in Tabelle 1 dargestellten Cyclohexenonverbindungen III lassen sich auf analogem Wege herstellen.

Tabelle 1: Cyclohexenonverbindungen III

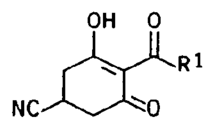

III

| Nr. | $R^1$ | phys. Daten (Fp [°C]; $^1$H-NMR [δ in ppm]) |
|---|---|---|
| 1.1 | Methyl | 94 bis 97 |
| 1.2 | Ethyl | 70 bis 74 |
| 1.3 | Propyl | 47 bis 50 |
| 1.4 | Butyl | 58 bis 59 |
| 1.5 | Pentadecanyl | 66 bis 69 |
| 1.6 | Cyclopropyl | 1,2 (m); 1,35 (m); 3,0 (m); 3,3 (m) |
| 1.7 | Cyclohexyl | |
| 1.8 | Phenyl | |
| 1.9 | 4-Cl, 2-$NO_2$-$C_6H_3$ | 184 bis 187 (Zers.) |

Beispiel 2.1

S-Methyl-3,5-dioxo-4-propionyl-cyclohexancarbonsäureimido-thioester hydrochlorid

5,0 g (0,026 Mol) 3,5-Dioxo-4-propionyl-cyclohexancarbonsäurenitril und 1,37 g (0,028 Mol) Methylmercaptan wurden in 100 ml trockenem Diethylether gelöst. Nach dem Abkühlen auf 0°C wurde ein kräftiger, trockener Chlorwasserstoffstrom durch die Lösung geleitet. Nach 0,5 h wurde der Gasstrom abgestellt und das Reaktionsgemisch langsam auf Raumtemperatur erwärmt. Erneut wurde auf 0°C abgekühlt, Chlorwasserstoff eingeleitet (0,5 h), danach die Kühlung entfernt und über Nacht stehen gelassen. Die abgeschiedenen Kristalle wurden abgesaugt und im Vakuum getrocknet: 5,4 g (74 % d. Th.) der Verbindung 2.1 [Fp. 161°C (Zers.)].

Beispiel 2.2

S-Ethyl-3,5-dioxa-4-propionyl-cyclohexancarbonsäureimido-thioester hydrochlorid

19,3 g (0,1 Mol) 3,5-Dioxo-4-propionyl-cyclohexancarbonsäurenitril und 6,5 g (0,105 Mol) Ethylmercaptan wurden in 800 ml trockenem Diethylether gelöst und wie in Beispiel 2.1 beschrieben mit Chlorwasserstoff umgesetzt. 20,7 g (71 % d. Th.) der Verbindung 2.2 [Fp. 180-185°C (Zers.)].

Beispiel 2.3

S-Ethyl-3,5-dioxo-4-palmitoyl-cyclohexancarbonsaureimido- thioester hydrochlorid

7,5 g (0,02 Mol) 3,5-Dioxo-4-palmitoyl-cyclohexancarbonsaurenitril und 1,86 g (0,03 Mol) Ethylmercaptan wurden in 150 ml trockenem Diethylether suspendiert. Nach dem Abkühlen auf 0°C wurde ein kräftiger, trockener Chlorwasserstoffstrom (konz. Schwefelsäure) durch die Suspension geleitet. Nach 0,5 h wird der Gasstrom abgestellt und das Reaktionsgemisch langsam auf Raumtemperatur erwärmt. Dann wurde erneut auf 0°C abgekühlt, Chlorwasserstoff eingeleitet, die Kühlung entfernt und nach insgesamt 6 h der Feststoff abfiltriert, mit Ether gewaschen und getrocknet: 7,3 g (77 % d. Th.) der Verbindung 2.3 [Fp. 65-68 (Zers.)].

Analog können die in Tabelle 2 aufgeführten Imidothioester V hergestellt werden.

Tabelle 2: Cyclohexantrioncarbonsäureimidothioester V

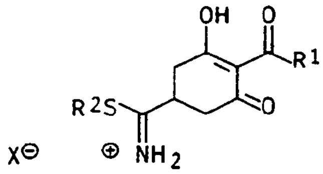

V

| Nr. | $R^1$ | $R^2$ | X | phys. Daten Fp (°C) |
|---|---|---|---|---|
| 2.1 | Ethyl | Methyl | Cl | 161 (Zers.) |
| 2.2 | Ethyl | Ethyl | Cl | 180 bis 185 (Zers.) |
| 2.3 | n-Pantadecanyl | Ethyl | Cl | 65-68 |
| 2.4 | Methyl | Ethyl | Cl | 160 bis 165 (Zers.) |
| 2.5 | Ethyl | 2-Methoxyethyl | Cl | öl |
| 2.6 | Cyclopropyl | 2-Methoxyethyl | Cl | öl |

Beispiel 3.1

S-Methyl-3,5-dioxo-4-propionyl-cyclohexancarbonsäurethiolester

2,8 g (0,01 Mol) der Verbindung 2.1 wurden in 50 ml 10 %iger Salzsäure gelöst und über Nacht bei Raumtemperatur gerührt. Der weipe Niederschlag wurde abfiltriert, mit Wasser gewaschen und im Vakuum getrocknet: 1,95 g (80 % d. Th.) der Verbindung 3.1 (Fp. 81°C).

Beispiel 3.2

S-Ethyl-3,5-dioxo-4-propionyl-cyclohexancarbonsäurethiolester

5,8 g (0,02 Mol) von Verbindung 2.2 wurden in 100 ml 10 %iger Salzsäure gelöst und über Nacht bei Raumtemperatur gerührt. Der weiße Niederschlag wurde abfiltriert, mit Wasser gewaschen und im Vakuum getrocknet: 4,1 g (80 % d. Th.) der Verbindung 3.2 (Fp. 70°C).

Beispiel 3.3

S-Ethyl-3,5-dioxo-4-palmitoyl-cyclohexancarbonsäurethiolester

3,0 g (6,3 mmol) der Verbindung 2.3 wurden in 50 ml Wasser suspendiert und über Nacht bei Raumtemperatur kräftig gerührt. Der Feststoff wurde abfiltriert, mit Wasser gewaschen und im Vakuum getrocknet: 2,4 g (86 % d. Th.) der Verbindung 3.3 (Fp. 56-58°C).

Die in der nachfolgenden Tabelle 3 aufgeführten Verbindungen I wurden analog hergestellt.

Tabelle 3: Cyclohexancarbonsäurethiolester I

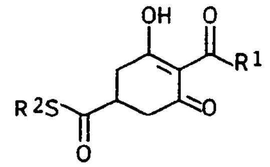

I

| Nr. | $R^1$ | $R^2$ | phys. Daten, Fp (°C) |
|---|---|---|---|
| 3.1 | Ethyl | Methyl | 81 |
| 3.2 | Ethyl | Ethyl | 70 |
| 3.3 | n-Pantadecanyl | Ethyl | 56 bis 58 |
| 3.4 | Methyl | Ethyl | |
| 3.5 | Ethyl | 2-Methoxyethyl | Öl |
| 3.6 | Cyclopropyl | Ethyl | Öl |
| 3.7 | Cyclopropyl | 2-Methoxyethyl | Öl |
| 3.8 | 4-Cl, 2-$NO_2$-$C_6H_3$ | Ethyl | Öl |

## Patentansprüche

1. Verfahren zur Herstellung von Cyclohexantrioncarbonsäurethiolestern der allgemeinen Formel I

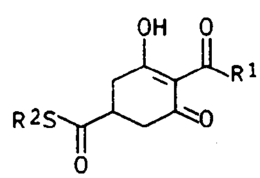

I

in der
$R^1$ für $C_1$-$C_{20}$-Alkyl, $C_2$-$C_{20}$-Alkenyl, $C_2$-$C_{20}$-Alkinyl oder $C_3$-$C_6$-Cycloalkyl, jeweils unsubstituiert oder durch Halogen, $C_1$-$C_4$-Alkyloxy, $C_1$-$C_4$-Alkylthio substituiert, Benzyl oder Phenyl jeweils unsubstituiert oder substituiert durch Halogen, Cyano, $C_1$-$C_4$-Alkyl, $C_1$-$C_4$-Alkoxy, $C_1$-$C_4$-Halogenalkyl oder Nitro und
$R^2$ für Wasserstoff, $C_1$-$C_6$-Alkyl, $C_2$-$C_6$-Alkenyl, $C_2$-$C_6$-Alkinyl und $C_3$-$C_6$-Cycloalkyl, jeweils unsubstituiert oder durch $C_1$-$C_4$-Alkoxy, $C_1$-$C_4$-Alkylthio, $C_1$-$C_4$-Dialkylamino, Hydroxy, Halogen substituiert; Benzyl oder Phenyl, jeweils unsubstituiert oder substituiert durch Halogen, Cyano, $C_1$-$C_4$-Alkyl, $C_1$-$C_4$-Alkoxy oder Nitro
stehen; dadurch gekennzeichnet, daß man in einer ersten Stufe eine Verbindung der allgemeinen Formel II

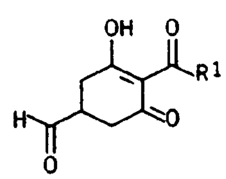

II

in der $R^1$ die oben angegebene Bedeutung hat, mit Hydroxylamin oder Hydroxylamin-O-sulfonsäure in einem inerten Lösungsmittel bei Temperaturen von 0 bis 150°C zu Verbindungen der allgemeinen Formel III

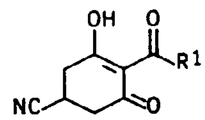

III,

in der $R^1$ die oben angegebene Bedeutung hat, umsetzt,
in einer zweiten Stufe diese Cyanoverbindungen der Formel III dann mit einem Mercaptan der allgemeinen Formel IV

$R^2$-SH IV

in der $R^2$ die oben angegebene Bedeutung hat, in Gegenwart einer wasserfreien Säure HX, zu einer Verbindung der allgemeinen Formel V

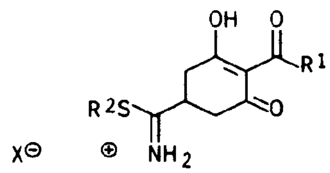

V,

in der $R^1$ und $R^2$ die oben angegebene Bedeutung haben und X für einen Säurerest steht, umsetzt und in einer dritten Stufe die Verbindungen der Formel V durch Hydrolyse in die Verbindungen der allgemeinen Formel I überführt.

**2.** Verfahren nach Anspruch 1, dadurch gekennzeichnet, daß man in der ersten Verfahrensstufe Verbindung I mit Hydroxylamin-O-sulfonsäure in homogener oder heterogener wäßriger Phase in einem pH-Bereich von 1 bis 9 umsetzt.

**3.** Verfahren nach den Ansprüchen 1 und 2, dadurch gekennzeichnet, daß man die Umsetzung mit Hydroxylamin-O-sulfonsäure in einem pH-Bereich von 3 bis 8 vornimmt.

**4.** Verfahren nach den Ansprüchen 1 bis 3, dadurch gekennzeichnet, daß die Umsetzungstemperatur in der ersten Verfahrensstufe 20 bis 80°C beträgt.

**5.** Verfahren nach Anspruch 1, dadurch gekennzeichnet, daß man als Säure in der zweiten Verfahrensstufe Chlorwasserstoff oder Bromwasserstoff verwendet.

**6.** Verfahren nach den Ansprüchen 1 und 5, dadurch gekennzeichnet, daß man die Umsetzung der zweiten Verfahrensstufe bei einer Temperatur von -20 bis 100°C vornimmt.

**7.** Verfahren nach Anspruch 1, dadurch gekennzeichnet, daß man die Hydrolyse der Verbindung V bei einem pH zwischen 1 und 7 und einer Temperatur von 0 bis 100°C durchführt.

**8.** Verfahren nach den Ansprüchen 1 bis 8, dadurch gekennzeichnet, daß man die Verfahrensstufen 1 bis 3 ohne Isolierung der Zwischenverbindungen III und V durchführt.

**9.** Cyclohexantrionverbindungen der allgemeinen Formel V

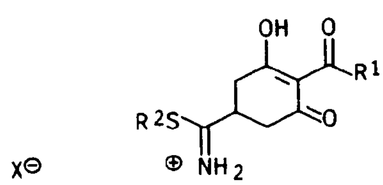

V,

in der

$R^1$ für $C_1$-$C_{20}$-Alkyl, $C_2$-$C_{20}$-Alkenyl, $C_2$-$C_{20}$-Alkinyl oder $C_3$-$C_6$-Cycloalkyl, jeweils unsubstituiert oder durch Halogen, $C_1$-$C_4$-Alkyloxy, $C_1$-$C_4$-Alkylthio substituiert, Benzyl oder Phenyl jeweils unsubstituiert oder substituiert durch Halogen, Cyano, $C_1$-$C_4$-Alkyl, $C_1$-$C_4$-Alkoxy, $C_1$-$C_4$-Halogenalkyl oder Nitro;

R² für Wasserstoff, $C_1$-$C_6$-Alkyl, $C_2$-$C_6$-Alkenyl, $C_2$-$C_6$-Alkinyl und $C_3$-$C_6$-Cycloalkyl, jeweils unsubstituiert oder durch $C_1$-$C_4$-Alkoxy, $C_1$-$C_4$-Alkylthio, $C_1$-$C_4$-Dialkylamino, Hydroxy, Halogen substituiert; Benzyl oder Phenyl, jeweils unsubstituiert oder substituiert durch Halogen, Cyano, $C_1$-$C_4$-Alkyl, $C_1$-$C_4$-Alkoxy oder Nitro und

X für einen Säurerest aus der Gruppe Chlorid, Bromid, Sulfat, Phosphat, Tetrafluoroborat, Perchlorat und Trifluoracetat

stehen.

**10.** Cyclohexantrionverbindungen der allgemeinen Formel V gemäß Anspruch 9, in der

R¹ für $C_1$-$C_4$-Alkyl, $C_3$-$C_6$-Cycloalkyl, $C_2$-$C_6$-Alkoxyalkyl, $C_2$-$C_6$-Alkylthioalkyl, Benzyl oder Phenyl unsubstituiert oder ein- bis dreifach substituiert durch Halogen, Cyano, $C_1$-$C_4$-Alkyl, $C_1$-$C_4$-Alkoxy, $C_1$-$C_4$-Halogenalkyl oder Nitro;

R² für $C_1$-$C_6$-Alkyl, $C_3$-$C_5$-Alkenyl, $C_3$-$C_4$-Alkinyl, $C_3$-$C_6$-Cycloalkyl, $C_2$-$C_6$-Alkoxyalkyl, $C_2$-$C_6$-Alkylthioalkyl, Benzyl oder Phenyl unsubstituiert oder ein- bis dreifach substituiert durch Halogen, Cyano, $C_1$-$C_4$-Alkyl, $C_1$-$C_4$-Alkoxy oder Nitro und

X für Chlorid oder Bromid

stehen.

## Claims

**1.** A process for preparing an acylcyclohexadionethiocarboxylic S-ester of the formula I

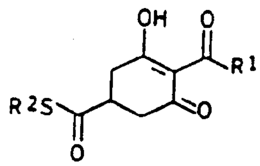

I

where

$R^1$ is $C_1$-$C_{20}$-alkyl, $C_2$-$C_{20}$-alkenyl, $C_2$-$C_{20}$-alkynyl or $C_3$-$C_6$-cycloalkyl, each of which is unsubstituted or substituted by halogen, $C_1$-$C_4$-alkoxy or $C_1$-$C_4$-alkylthio, or is benzyl or phenyl, each of which is unsubstituted or substituted by halogen, cyano, $C_1$-$C_4$-alkyl, $C_1$-$C_4$-alkoxy, $C_1$-$C_4$-halo-alkyl or nitro, and

$R^2$ is hydrogen, $C_1$-$C_6$-alkyl, $C_2$-$C_6$-alkenyl, $C_2$-$C_6$-alkynyl or $C_3$-$C_6$-cycloalkyl, each of which is unsubstituted or substituted by $C_1$-$C_4$-alkoxy, $C_1$-$C_4$-alkylthio, $C_1$-$C_4$-di-alkylamino, hydroxyl or halogen; benzyl or phenyl, each of which is unsubstituted or substituted by halogen, cyano, $C_1$-$C_4$-alkyl, $C_1$-$C_4$-alkoxy or nitro,

which comprises reacting, in a first stage, a compound of the formula II

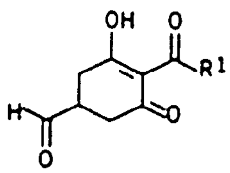

II

where $R^1$ has the abovementioned meaning, with hydroxylamine or hydroxylamine-O-sulfonic acid in an inert solvent at from 0 to 150°C to give a compound of the formula III

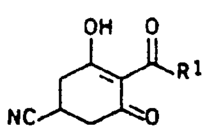

III

where $R^1$ has the abovementioned meaning,

and then, in a second stage, reacting this cyano compound of the formula III with a mercaptan of the formula IV

$R^2$-SH IV

where $R^2$ has the abovementioned meaning, in the presence of an anhydrous acid HX, to give a compound of the formula V

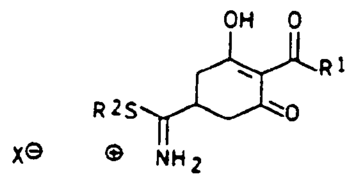

V

where $R^1$ and $R^2$ have the abovementioned meanings, and X is the anion of an acid, and, in a third stage, hydrolyzing the compound of the formula V to give the compound of the formula I.

**2.** A process as claimed in claim 1, in the first stage of which compound I is reacted with hydroxylamine-O-sulfonic acid in homogeneous or heterogeneous aqueous phase at a pH of from 1 to 9.

**3.** A process as claimed in claim 1 or 2, wherein the reaction with hydroxylamine-O-sulfonic acid is carried out at a pH of from 3 to 8.

**4.** A process as claimed in any of claims 1 to 3, wherein the reaction in the first stage is carried out at from 20 to 80°C.

**5.** A process as claimed in claim 1, wherein the acid used in the second stage is hydrogen chloride or hydrogen bromide.

**6.** A process as claimed in claim 1 or 5, wherein the reaction in the second stage is carried out at from -20 to 100°C.

**7.** A process as claimed in claim 1, wherein the hydrolysis of compound V is carried out at a pH of from 1 to 7 and at from 0 to 100°C.

**8.** A process as claimed in any of claims 1 to 8, wherein stages 1 to 3 are carried out without isolation of the intermediates III and V.

**9.** An acylcyclohexadione of the formula V

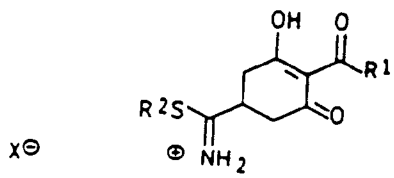

V

where

$R^1$ is $C_1$-$C_{20}$-alkyl, $C_2$-$C_{20}$-alkenyl, $C_2$-$C_{20}$-alkynyl or $C_3$-$C_6$-cycloalkyl, each of which is unsubstituted or substituted by halogen, $C_1$-$C_4$-alkoxy or $C_1$-$C_4$-alkylthio, or is benzyl or phenyl, each of which is unsubstituted or substituted by halogen, cyano, $C_1$-$C_4$-alkyl, $C_1$-$C_4$-alkoxy, $C_1$-$C_4$-haloalkyl or nitro;

$R^2$ is hydrogen, $C_1$-$C_6$-alkyl, $C_2$-$C_6$-alkenyl, $C_2$-$C_6$-alkynyl or $C_3$-$C_6$-cycloalkyl, each of which is unsubstituted or substituted by $C_1$-$C_4$-alkoxy, $C_1$-$C_4$-alkylthio, $C_1$-$C_4$-dialkylamino, hydroxyl or halogen; benzyl or phenyl, each of which is unsubstituted or substituted by halogen, cyano, $C_1$-$C_4$-alkyl, $C_1$-$C_4$-alkoxy or nitro, and

X is a chloride, bromide, sulfate, phosphate, tetrafluoroborate, perchlorate or trifluoroacetate ion.

**10.** An acylcyclohexadione of the formula V as claimed in claim 9, where

$R^1$ is $C_1$-$C_4$-alkyl, $C_3$-$C_6$-cycloalkyl, $C_2$-$C_6$-alkoxyalkyl, $C_2$-$C_6$-alkylthioalkyl, benzyl or phenyl unsubstituted or substituted once to three times by halogen, cyano, $C_1$-$C_4$-alkyl, $C_1$-$C_4$-alkoxy, $C_1$-$C_4$-haloalkyl or nitro;

$R^2$ is $C_1$-$C_6$-alkyl, $C_3$-$C_5$-alkenyl, $C_3$-$C_4$-alkynyl, $C_3$-$C_6$-cycloalkyl, $C_2$-$C_6$-alkoxyalkyl, $C_2$-$C_6$-alkylthioalkyl, benzyl or phenyl unsubstituted or substituted once to three times by halogen, cyano, $C_1$-$C_4$-alkyl, $C_1$-$C_4$-alkoxy or nitro, and

X is chloride or bromide.

## Revendications

1. Procédé pour préparer des esters de thiols et d'acides cyclohexanetrionecarboxyliques répondant à la formule générale I

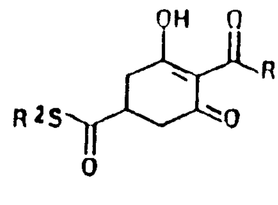

I

dans laquelle

$R_1$ est un radical alkyle en $C_1$-$C_{20}$, alcényle en $C_2$-$C_{20}$, alcinyle en $C_2$-$C_{20}$ ou cycloalkyle en $C_3$-$C_6$, qui chaque fois sont non substitués ou substitués par des substituants halogéno, alkyloxy en $C_1$-$C_4$ ou alkylthio en $C_1$-$C_4$ ; benzyle ou phényle, chaque fois non substitués ou substitués par des substituants halogéno, cyano, alkyle en $C_1$-$C_4$, alcoxy en $C_1$-$C_4$, halogénalkyle en $C_1$-$C_4$ ou nitro, et

$R_2$ est un hydrogène ou un radical alkyle en $C_1$-$C_6$, alcényle en $C_2$-$C_6$, alcinyle en $C_2$-$C_6$ ou cycloalkyle en $C_3$-$C_6$, chaque fois non substitués ou substitués par des substituants alcoxy en $C_1$-$C_4$, alkylthio en $C_1$-$C_4$, dialkylamino en $C_1$-$C_4$, hydroxy, halogéno ; benzyle ou phényle, chaque fois non substitués ou substitués par des substituants halogéno, cyano, alkyle en $C_1$-$C_4$, alcoxy en $C_1$-$C_4$ ou nitro ;

caractérisé en ce que, dans une première étape, on fait réagir un composé répondant à la formule générale II

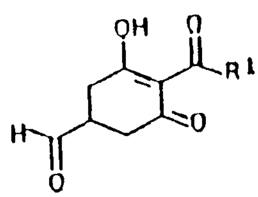

II

dans laquelle $R_1$ a les significations données ci-dessus, avec de l'hydroxylamine ou de l'acide hydroxylamine-O-sulfonique dans un solvant inerte à des températures comprises entre 0 et 150°C, pour obtenir des composés répondant à la formule générale III

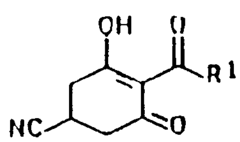

III,

dans laquelle $R_1$ a les significations données ci-dessus ;

dans une deuxième étape, on fait réagir ces composés cyano de formule III avec un mercaptan répondant à la formule générale IV

$R^2$-SH IV

dans laquelle $R^2$ a les significations donnés ci-dessus, en présence d'un acide anhydre HX pour obtenir un composé répondant à la formule générale V

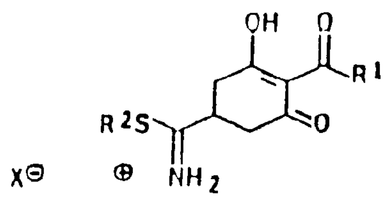

V,

dans laquelle $R^1$ et $R^2$ a les significations données ci-dessus et X est un résidu d'acide, et dans une troisième étape, on convertit par hydrolyse les composés répondant à la formule V en les composés répondant à la formule générale I.

2. Procédé selon la revendication 1, caractérisé en ce que, dans la première étape, on fait réagir le composé I avec de l'acide hydroxylamine-O-sulfonique en phase aqueuse homogène ou hétérogène à pH compris entre 1 et 9.

3. Procédé selon les revendications 1 et 2, caractérisé en ce qu'on procède à la réaction avec l'acide hydroxylamine-O-sulfonique à un pH compris entre 3 et 8.

4. Procédé selon les revendications 1 à 3, caractérisé en ce que la température de la réaction est comprise entre 20 et 80°C lors de la première étape.

5. Procédé selon la revendication 1, caractérisé en ce qu'on utilise comme acide, dans la deuxième étape, de l'acide chlorhydrique ou l'acide bromhydrique.

6. Procédé selon les revendications 1 et 5, caractérisé en ce que la réaction de la deuxième étape est mise en oeuvre à une température de -20 à 100°C.

7. Procédé selon la revendication 1, caractérisé en ce qu'on procède à l'hydrolyse du composé V à un pH compris entre 1 et 7 et à une température comprise entre 0 et 100°C.

8. Procédé selon les revendications 1 à 8, caractérisé en ce qu'on met en oeuvre les étapes 1 à 3 sans isoler les composés intermédiaires III et V.

9. Cyclohexanetrione répondant à la formule générale V

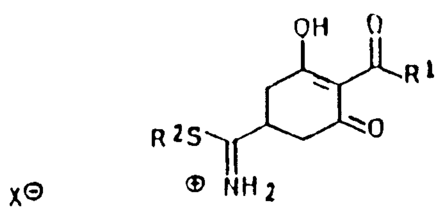

V,

dans laquelle
$R_1$ est un radical alkyle en $C_1$-$C_{20}$, alcényle en $C_2$-$C_{20}$, alcinyle en $C_2$-$C_{20}$ ou cycloalkyle en $C_3$-$C_6$, qui chaque fois sont non substitués ou substitués par des substituants halogéno, alkyloxy en $C_1$-$C_4$ ou alkylthio en $C_1$-$C_4$ ; benzyle ou phényle, chaque fois non substitués ou substitués par des substituants halogéno, cyano, alkyle en $C_1$-$C_4$, alcoxy en $C_1$-$C_4$, halogénalkyle en $C_1$-$C_4$ ou nitro, et
$R_2$ est un hydrogène ou un radical alkyle en $C_1$-$C_6$, alcényle en $C_2$-$C_6$ , alcinyle en $C_2$-$C_6$ ou cycloalkyle en $C_3$-$C_6$, chaque fois non substitués ou substitués par des substituants alcoxy en $C_1$-$C_4$, alkylthio en $C_1$-$C_4$, dialkylamino en $C_1$-$C_4$, hydroxy, halogéno ; benzyle ou phényle, chaque fois non substitués ou substitués par des substituants halogéno, cyano, alkyle en $C_1$-$C_4$, alcoxy en $C_1$-$C_4$ ou nitro ; et
X est un résidu d'acide choisi parmi l'ensemble comprenant le chlorure, le bromure, le sulfate, le phosphate, le tétrafluoroborate, le perchlorate et le trifluoracétate.

10. Cyclohexanetriones répondant à la formule générale V selon la revendication 9, dans laquelle
$R^1$ est un radical alkyle en $C_1$-$C_4$, cycloalkyle en $C_3$-$C_6$, alcoxyalkyle en $C_2$-$C_6$, alkylthioalkyle en $C_2$-$C_6$, benzyle ou phényle non substitué ou une à trois fois substitué par des substituants halogéno, cyano, alkyle

en $C_1$-$C_4$, alcoxy en $C_1$-$C_4$, halogénalkyle en $C_1$-$C_4$ou nitro ;
$R^2$ est un radical alkyle en $C_1$-$C_6$, alcényle en $C_3$-$C_5$, alcinyle en $C_3$-$C_4$, cycloalkyle en $C_3$-$C_6$, alcoxyalkyle en $C_2$-$C_6$, alkylthioalkyle en $C_2$-$C_6$, benzyle ou phényle non substitué ou une à trois fois substitué par des substituants halogéno, cyano, alkyle en $C_1$-$C_4$, alcoxy en $C_1$-$C_4$ou nitro et
X est le chlorure ou le bromure.